# EUROPÄISCHE PATENTSCHRIFT

(11) **EP 4 574 103 B1**
(45) Veröffentlichungstag und Bekanntmachung des Hinweises auf die Patenterteilung: **18.03.2026**
(21) Anmeldenummer: 23020557.7
(22) Anmeldetag: 18.12.2023
(51) Int. Cl.: A61F 5/01

(54) **SPRUNGGELENKORTHESE**
ANKLE JOINT OR ANKLE JOINT
ORTHÈSE DE CHEVILLE

(43) Veröffentlichungstag der Anmeldung: 25.06.2025
(73) Patentinhaber: medi GmbH & Co. KG, 95448 Bayreuth (DE)
(72) Erfinder: Poetzschner, Felix, 95445 Bayreuth (DE); Wagner, Harald, 83080 Oberaudorf (DE); Zellnitz, Stefan, 6071 Aldrans (AT); Meinhardt, Maximilian, 6020 Innsbruck (AT)

(56) Entgegenhaltungen:
- WO-A1-87/02885
- US-A1- 2002 029 009
- US-A1- 2006 178 606
- US-A1- 2019 015 234
- US-B2- 8 202 239

## Beschreibung

Die vorliegende Erfindung bezieht sich auf eine Sprunggelenkorthese nach dem Oberbegriff des Anspruch 1.

Derartige Sprunggelenkorthesen dienen zum Stützen, Entlasten und Ruhigstellen des Fußes eines Trägers, insbesondere bei Verstauchungen, Sprunggelenkbrüchen, Schmerzen an Sehnen und Bändern im Bereich des Sprunggelenks, oder nach Operationen. Dabei wird durch die Orthese verhindert, dass der Träger den Fuß, insbesondere das Sprunggelenk, seitlich abwinkeln und/ oder nach oben oder unten beugen kann. Zudem dienen derartige Orthesen aber auch dazu, Schwellungen, insbesondere Ödeme und Hämatome, abzubauen.

Diese Orthesen für das Sprunggelenk bestehen dabei üblicherweise aus einem textilen, vorzugsweise kompressiven, Grundkörper, insbesondere um den Abbau von Schwellungen zu fördern, der in seiner Form der Fußanatomie im Bereich des Sprunggelenks angepasst ist. Dieser Grundkörper umgibt hierbei in der Tragstellung das Sprunggelenk und die angrenzenden Bereiche des Fußes bevorzugt an allen Seiten. Zur Stabilisierung und Ruhigstellung ist es ferner bekannt, dass die Sprunggelenkorthesen neben dem textilen Grundkörper ein Stabilisierungselement aufweisen, welches bevorzugt mit dem Grundkörper verbunden ist. Das Stabilisierungselement verhindert das seitliche Abwinkeln und/ oder das Beugen des Fußes. Mittels eines Gurtsystems, welches bevorzugt an dem Grundkörper oder dem Stabilisierungselement angebracht ist, wird der Grundkörper und das Stabilisierungselement fest an das Sprunggelenk angelegt und dort fixiert.

Eine derartige Orthese, insbesondere zur Stabilisierung des Sprunggelenks, ist beispielsweise aus der EP 2 666 445 B1 bekannt.

Dabei umfasst die Orthese zur Immobilisierung des Sprunggelenks ein Gestrickteil, das der Anatomie des Fußes im Bereich des Sprunggelenks angepasst gestrickt ist und dieses in der Tragstellung allseitig umfasst. Dieses kann zum Anlegen der Orthese abschnittsweise geöffnet oder geweitet werden. Durch ein Verschlusssystem, insbesondere in Form eines Schnürsenkels mit mehreren Haken und Ösen entlang einer Öffnung zum Einsteigen in die Orthese, kann diese, insbesondere der Gestrickkörper, zusammengezogen und gespannt werden.

Ferner weist die Orthese ein, vorzugsweise einteiliges, Stabilisierungselement auf, das einen Fersenabschnitt, einen Wadenabschnitt sowie einen die beiden Abschnitte verbindenden Seitenabschnitt umfasst. An der Außenseite des Stabilisierungselements sowie an der Innenseite des Gestrickteils sind nun Verbindungselemente vorgesehen, um beide Elemente lösbar miteinander zu fixieren.

Ebenfalls ist ein Gurt vorgesehen, der an dem Gestrickteil befestigt werden kann. Dieser Gurt dient zur weitergehenden Immobilisierung des Sprunggelenks in der Tragstellung. Der Gurt ist in einer 8er-Zügelung um das Sprunggelenk geführt. Dabei verläuft der Gurt von der lateralen Seite über den Spann zur medialen Seite. Von dort wird er unter der Fußsohle eines Trägers erneut nach lateral geführt und erstreckt sich erneut von dort über den Spann nach medial. Dadurch kann eine nahezu vollständige Immobilisierung des Fußgelenks erreicht werden.

Eine weitere Sprunggelenkorthese, insbesondere in Form einer Sprunggelenkschiene, ist beispielsweise aus der EP 2 050 429 A1 bekannt.

Die Orthese für das Sprunggelenk umfasst dabei ein das Sprunggelenk eines Patienten stützendes Element, welches aus einem medialen und lateralen Seitenteil, die an der Seite des Unterschenkels angelegt werden, besteht. Über ein Sohlenteil, das unterhalb der Fußsohle eines Patienten verläuft, sind beide Seitenteile miteinander verbunden. Im Tragezustand wird das Stützelement mittels eines ersten oberen Gurtes, der zirkulär um beide Seitenteile herumgeschlungen wird, am Bein des Patienten befestigt. Der obere Schalengurt befindet sich somit im oberen Endbereich der beiden Seitenteile. Dort ist er mit einem Ende fest mit einem der Seitenteile verbunden. Sein freies Ende wird mittels eines Klettverschlusses auf sich selbst befestigt.

Neben dem oberen zirkulär verlaufenden Gurt weist nun die Orthese einen zweiten sogenannten Pronationsgurt auf, um den Vorfuß eines Patienten anzuheben, um insbesondere die Außenbänder zu entlasten. Dieser verläuft nicht nur zirkulär, wie der obere Gurt, um die beiden Seitenteile. Der Gurt weist vielmehr einen quer unter der Fußsohle entlangführbaren Sohlenabschnitt und einen daran anschließenden über die laterale Außenseite des Fußes und den Fußrücken führbaren Lateral-/Ristabschnitt auf. Beim Anlegen wird der Gurt an der lateralen Seite befestigt und über den Spann nach medial geführt. Von dort erstreckt sich der Gurt unterhalb der Fußsohle erneut auf die laterale Seite. Anschließend wird er erneut über den Spann und zwar unterhalb des ersten Gurtabschnittes nach medial geführt. Von dort schließlich wird dieser einmal zirkulär um den Fuß des Patienten geführt und medial befestigt.

Ferner weist, der Pronationsgurt ein Fluidpolsterkissen auf, das beim Gehen einen dynamischen Pumpeffekt auf die anliegenden Fußbereiche ausübt und damit als Massagekissen wirkt.

Auch aus der US 2006178606 A1 ist eine Orthese für das Sprunggelenk bekannt. Die Sprunggelenkorthese umfasst dabei ebenfalls einen Grundkörper mit einem ersten und zweiten Seitenteil sowie einem unteren Teil, welcher unterhalb der Fußsohle eines Bnutzers zum Liegen kommt. Ein Riemen, welcher an einem Seitenabschnitt der Orthese befestigt ist, erstreckt sich dabei von der medialen Seite der Orthese in Richtung laterale Seite und dient dazu den Vorfuß eines Benutzers anzuheben, wenn dieser Riemen festgezogen wird. Dabei wird der Riemen an der lateralen Seite der Orthese durch eine Gurtdurchführung geführt, woraufhin dieser dann entlang der Rückseite der Orthese und über das Schienbein geführt wird. Anschließend kann der Riemen an einem Befestigungselement an der lateralen Seite der Orthese befestigt werden. Ferner weist die Orthese bevorzugt eine aufblasbare Zelle auf, die dazu ausgebildet ist, den Fuß des Benutzers zu stützen.

Die US 8202239 B2 offenbart eine Orthese zur Stabilisierung des Sprunggelenks mit einem Grundkörper und ebenso mit einem Gurtsystem. Der Grundkörper ist dabei als halbstarre Manschette ausgebildet und umschließt den Unterschenkel. Das Gurtsystem wiederum umfasst bevorzugt zwei schwenkbare Haltegurte, welche an der lateralen Seite der Orthese angeordnet und dort befestigt sind. Zudem ist ein weiterer Gurt, insbesondere ein Derotationsgurt, an der Orthese, insbesondere ebenfalls an deren lateralen Seite, befestigt, welcher die Orthese vollständig umläuft und schließlich erneut an der lateralen Seite befestigt wird. Neben dem Gurtsystem wird ein Schnürverschlussmechanismus offenbart, der die seitlichen Abschnitte der Orthese verbindet.

Auch aus der US 2002029009 A1 ist eine therapeutische Knöchel- und Fußorthese bekannt. Diese besteht im Wesentlichen aus zwei Schalenteilen, insbesondere einer seitlichen Schale sowie einer Fußschale, welche sich ebenfalls nach oben erstreckt, um die seitliche Schale zu überlappen. Beide Schalenteile sind schwenkbar miteinander verbunden, dies insbesondere über eine Schwenkeinrichtung. Ferner erstreckt sich von der Fußschale ein zweiter Seitenabschnitt. Neben den mehreren Schalenteilen umfasst die Orthese auch eine Riemeneinrichtung zum Halten der Schalen am Fuß des Benutzers. Die Riemenanordnung umfasst dabei zwei vorderseitig über den Spann eines Trägers verlaufende Gurte sowie einen rückseitig um den Fuß eines Trägers verlaufenden und ebenfalls die beiden seitlichen Schalenteile verbindenden Gurt.

Die aus der US 2019015234 A1 bekannte Orthese für ein Fußgelenk umfasst neben einem hülsenartigen Grundkörper ein Gurtsystem, insbesondere zum Stützen der Schienbeinsehnen. Das Gurtsystem weist mehrere Gurtabschnitte auf. Zwei erste Abschnitte sind an der Rückseite des Grundkörpers befestigt und verlaufen jeweils seitlich am Fuß eines Trägers vorbei in einen vorderen Bereich der Orthese. Dort überlappen die beiden Gurtabschnitte und das Gurtsystem kann so geschlossen werden. Unterhalb der zuvor genannten Abschnitte weist das Gurtsystem zwei weitere Gurtabschnitte auf, die diagonal über einen Spannbereich eines Trägers verlaufen und sich in diesem Bereich kreuzen. Der erste diagonale Gurt umläuft anschließend einen Fersenbereich eines Fußes eines Trägers und wird erneut seitlich auf sich selbst befestigt. Ein zweiter diagonaler Gurt verläuft, bevor er den Spannbereich kreuzt, ebenfalls unterhalb der Fußsohle, jedoch in einem Mittelfußbereich.

Schließlich offenbart auch die WO 8702885 A1 eine Fußgelenkstütze mit einem Grundkörper sowie einem daran angeordneten Gurtsystem zum Stützen des Fußgelenks. Das Gurtsystem weist dabei einen ersten Riemen auf, welche sich um die gesamte Orthese und um das Bein oberhalb des Knöchels eines Trägers erstreckt. Zwei weitere sehr kurze Riemen erstrecken sich nur seitlich entlang der Orthese. Diese entsprechen einigen der natürlichen Bänder des Sprunggelenks. Ein weiterer Riemen ist an der Unterseite der Orthese befestigt. Dieser Riemen verläuft unter dem Fuß nach oben entlang der Innenseite des Fußes und über die Vorderseite des Sprunggelenks. Durch die Einstellung des Zugverhältnisses der mehreren Riemen wird der Fuß gestützt und die seitliche Bewegung des Sprunggelenks begrenzt.

Als nachteilig dieser Ausgestaltungen der Sprunggelenkorthesen erweist sich, insbesondere bei Ausbildung der Orthesen mit einem Grundkörper, dass diese über ein zusätzliches Verschlusssystem geschlossen werden, um an dem Fuß eines Patienten angelegt und dort fixiert werden zu können. Mit der Verarbeitung und Anbringung der mehreren Komponenten des Verschlusssystems an dem Grundkörper, insbesondere der Haken und Ösen sowie des Schnürsenkels, ist ein erhöhter Materialverbrauch verbunden. Dies führt dazu, dass diese Art und Weise der Ausbildung der Orthese, insbesondere des Grundkörpers, sehr kostenintensiv ist. Dies auf Grund des zuvor genannten erhöhten Materialverbrauchs, aber insbesondere auch auf Grund des erhöhten Fertigungsaufwands.

Nachteilig ist ebenfalls, dass die bekannten Orthesen mit den textilen Grundkörpern nur schwer anzulegen sind. Um die Orthese anlegen zu können, muss der Grundkörper geweitet bzw. geöffnet werden, damit der Patient in die Orthese einsteigen kann. Dieses Öffnen des Grundkörpers, aber auch und insbesondere des Schnürsystems, ist nur mit Kraftaufwand möglich, wie es auch bei dem Einsteigen in einen Schuh der Fall ist. Anschließend muss der Grundkörper noch geschlossen werden, also das Schnürsystem zusammengezogen werden. Diese Schritte sind aufwendig und stellen für Personen, die alters- oder verletzungsbedingt Schwierigkeiten haben in den Fußbereich zu gelangen, eine besondere Herausforderung dar. Zudem ist zu berücksichtigen, dass im Anschluss als zusätzlicher Schritt noch das Gurtsystem angelegt werden muss.

Als nachteilig erweist sich dabei, insbesondere bezüglich des zusätzlichen Gurtsystems, dass dieses als separates Bauteil ausgebildet ist. Dadurch wird der Material- und Fertigungsaufwand erhöht, was ebenfalls zu erhöhten Produktionskosten führt. Das Gurtsystem muss zusätzlich zu dem Gurtkörper gefertigt und der Orthese beigelegt werden. Zudem bringt das bekannte Gurtsystem aber auch den Nachteil mit sich, dass dieses unterhalb der Fußsohle entlang verläuft, was den Tragekomfort der Orthese negativ beeinflusst. Auch ist das Gurtsystem dadurch und aus den zuvor genannten Gründen, für diverse Patientengruppen nur äußerst schwer anzulegen.

Als nachteilig erweist sich bei den bekannten Sprunggelenkorthesen, welche in Form von Sprunggelenkschienen ausgebildet sind, dass diese, auch wenn sie zwar durch die Stabilisierungselemente ein Stützen und Stabilisieren des Sprunggelenks ermöglichen, durch den fehlenden textilen Grundkörper, welcher bspw. in Form einer Socke ausgebildet ist, nicht dazu beitragen Schwellungen, insbesondere Ödeme und Hämatome, abzubauen.

Ebenfalls als Nachteil erweist sich die Anordnung des Gurtsystems an den Sprunggelenkschienen. Es ist üblich, dass diese unterhalb der Fußsohle entlang verlaufen, was den Tragekomfort der Orthese negativ beeinflusst. Dies hat, wie bereits zuvor erwähnt, zur Folge, dass das Gurtsystem und somit die Orthese dadurch für die Patienten nur schwer anzulegen ist.

Schließlich ergibt sich aber mit der Ausbildung der Orthesen mit einem separaten Gurtsystem auch der Nachteil, dass das Risiko besteht, dass ein Patent das Gurtsystem falsch anlegt, da durch die lose Ausbildung des Gurtsystems eine Vielzahl von Möglichkeiten bestehen, den Gurt am Fuß des Patienten zu positionieren. Die Sprunggelenkorthese wird dadurch nicht ordnungsgemäß am Fuß des Patienten fixiert. Dies hat wiederum zur Folge, dass der Fuß nur unzureichend gestützt, entlastet, ruhiggestellt und/ oder stabilisiert wird. Die teilweise komplexen Gurtanordnungen und Gurtverläufe um das Sprunggelenk herum stellen für den Patienten jedenfalls eine zusätzliche Herausforderung dar.

Der vorliegenden Erfindung liegt daher die Aufgabe zugrunde eine Sprunggelenkorthese bereit zu stellen, welche die Nachteile aus dem Stand der Technik vermeidet, welche also insbesondere einfach anzulegen ist, einfach und kostengünstig in der Herstellung ist und gleichzeitig die Stabilisierung des Sprunggelenks sowie den Abbau von Schwellungen im Vergleich zu den aus dem Stand der Technik bekannten Lösungen weiter verbessert.

Gemäß einem Ausführungsbeispiel besteht die erfindungsgemäße Sprunggelenkorthese aus einem Grundkörper, der den Fuß eines Trägers zumindest medial, lateral und posterior sowie plantar umgibt, sowie zumindest aus zwei Gurte, die sich von der medialen Seite des Grundkörpers erstrecken und zumindest anterior über den Fuß des Trägers verlaufen, wobei der Grundkörper an der lateralen Seite zumindest ein erstes Befestigungselement für den ersten Gurt und zumindest ein Umlenkelement für den zweiten Gurt aufweist, wobei der erste Gurt in seiner Länge derart ausgebildet ist, dass ein erster Abschnitt des Gurtes sich von medial nach lateral erstreckt und ein zweiter Abschnitt den Fuß eines Trägers posterior und anschließend anterior umläuft, so dass ein an seinem freien Ende an der Innenseite angebrachtes zweites Befestigungselement an dem ersten lateralen Befestigungselement des Grundkörpers befestigbar ist, und wobei der zweite Gurt der aus einem im Wesentlichen unelastischen Textilmaterial besteht an seinem freien Ende an der Außenseite zumindest ein drittes Befestigungselement aufweist, so dass dieser nach einer lateralen Umlenkung an der medialen Seite über ein viertes Befestigungselement auf sich selbst, an dem Grundkörper oder an dem ersten Gurt befestigbar ist.

Die anatomischen Hauptrichtungen anterior, posterior, plantar sowie medial und lateral dienen zur Beschreibung der Lage und des Verlaufs der einzelnen Komponenten der Sprunggelenkorthese im Tragezustand, also wenn die Orthese ordnungsgemäß am Fuß eines Trägers angelegt ist. Dabei wird mit "medial" die Innenseite, mit "lateral" die Außenseite, mit "anterior" die Vorderseite, mit "posterior" die und Rückseite des Fußes und mit "plantar" die Fußunterseite bezeichnet.

Ferner wird mit den Angaben "Innenseite" und "Außenseite" auf die zum Fuß gerichtete Seite (Innenseite) und vom Fuß eines Trägers abgewandte Seite (Außenseite) Bezug genommen.

Die mehreren Befestigungselemente sind hierbei bevorzugt aus mehreren Klettverbindungen gebildet, welche jeweils aus zwei Klettelementen bestehen, wovon eines flexible Widerhäkchen und das andere Schlaufen aufweist. Dabei besteht bevorzugt das erste und vierte Befestigungselement aus dem schlaufenförmigen Klettelement. Das zweite und dritte Befestigungselement hingegen aus dem Gegenklett mit den Widerhäkchen. Die mehreren Klettelemente sind dabei bevorzugt auf den Grundkörper und auf den ersten und zweiten Gurt aufgenäht. Alternativ ist es auch denkbar, dass diese aufgeklebt oder aufgeschweißt sind.

Dabei ist das Umlenkelement bevorzugt in Form eines D-Ringes ausgebildet, welcher direkt oder über ein Textilelement, insbesondere über eine Textillasche, an dem Grundkörper befestigt ist. Besonders bevorzugt ist dieser aus einem Kunststoff gefertigt und direkt an dem Grundkörper aufgenäht. Hierzu weist der D-Ring neben einem Umlenkabschnitt ferner einen davon abstehenden Befestigungsabschnitt auf, welcher bevorzugt mit dem Umlenkabschnitt einteilig ausgebildet ist. Der Befestigungsabschnitt weist nun, damit dieser mit dem Grundkörper vernähbar ist, einen vorzugsweise nut-förmigen Rücksprung auf. Material und Materialstärke des Befestigungsabschnittes sind nun im Bereich des Rücksprungs bevorzugt so gewählt, dass dieser von einer Nähmaschine, insbesondere von deren Nähnadel, durchdringbar ist, so dass das Umlenkelement über einen Faden im Bereich des Rücksprungs mit dem Grundkörper verbindbar ist.

Im getragenen Zustand der Sprunggelenkorthese verläuft somit der erste Gurt, insbesondere dessen erster Gurtabschnitt, beginnend an der medialen Seite des Grundkörpers auf Höhe des Innenknöchels über den Spann zur lateralen Seite des Grundkörpers oberhalb des Außenknöchels. Von dort aus umläuft der zweite Abschnitt des ersten Gurtes die Außenseite des Grundkörpers und somit den Fuß eines Trägers, bevorzugt auf Höhe der Fessel, vollständig und wird dann mit seinem freien Ende an der lateralen Seite an dem Grundkörper oberhalb des dort verlaufenden ersten Gurtabschnitts befestigt.

Hierdurch wird durch den Grundkörper und insbesondere durch den zuvor beschriebenen speziellen Verlauf des ersten Gurtes zum einen eine Stabilisierung des Sprunggelenks gewährleistet. Zum anderen und insbesondere wird dadurch aber auch der Abbau bzw. die Einschränkung von Schwellungen, insbesondere von Ödemen, unterstütz. Durch den ersten Gurt wird nämlich Kompression auf den Fuß, insbesondere auf einen Fessel- und Spannbereich ausgeübt. Dabei verläuft der erste Gurt insbesondere derart um den Fuß eines Trägers, dass keine Lücken zwischen dem Grundkörper und dem Gut entstehen, was die Bildung von Fensterödemen zusätzlich verhindert. Hierzu weist der erste Gurt eine Breite von mind. 5 cm, bevorzugt von mind. 6 cm, auf.

Der zweite Gurt, insbesondere ein sogenannter Talusgurt, erstreckt sich ebenfalls von der medialen Seite des Grundkörpers, bevorzugt von unterhalb des Innenknöchels, über den Spann des Fußes eines Trägers. Er wird dann auf der lateralen Seite des Grundkörpers in einem Mittelfußbereich über das an dem Grundkörper angebrachte Umlenkelement umgelenkt. Nach der Umlenkung verläuft dieser von der lateralen Seite des Grundkörpers, insbesondere von unterhalb des Außenknöchels, erneut über den Spann und wird dann auf der medialen Seite des Grundkörpers auf sich selbst, an dem Grundkörper oder an dem ersten Gurt, bevorzugt oberhalb des Innenknöchels im Bereich der Fessel, befestigt. Dadurch weist der zweite Gurt eine medial gerichtete Zugrichtung auf, was eine Zugwirkung auf die Außenkante des Fußes bewirkt. Dadurch wird ein Supinationsschutz für das Sprunggelenk des Trägers gebildet. Der zweite Gurt mindert also die Supinationsmöglichkeit des Fußes eines Trägers. Er weist dabei eine Breite von mind. 2 cm, bevorzugt von mind. 3 cm, auf.

Gemäß einem zweiten Ausführungsbeispiel weist die erfindungsgemäße Sprunggelenkorthese, insbesondere deren Grundkörper, an der lateralen Seite ein fünftes Befestigungselement und der erste Gurt an seiner Innenseite an dem Ende des ersten Gurtabschnitts ein sechstes Befestigungselement auf, so dass der erste Gurt an der lateralen Seite des Grundköpers befestigbar ist, bevor dieser den Fuß eines Trägers posterior umläuft und erneut lateral an dem ersten Befestigungselement fixiert wird.

Gemäß einem dritten Ausführungsbeispiel ist die erfindungsgemäße Sprunggelenkorthese, insbesondere deren Grundkörper, aus einem im Wesentlichen unelastischen Textilmaterial gefertigt. Dabei ist der Grundkörper bevorzugt aus einem Textillaminat gefertigt. Dieses umfasst dabei bevorzugt neben einer inneren Gewebelage, eine Schaumstofflage sowie eine äußere vorzugsweise im Wesentlichen unelastische Kunststofflage, insbesondere Polyurethanlage.

Gemäß einem weiteren Ausführungsbeispiel weist der Grundkörper der erfindungsgemäßen Sprunggelenkorthese an der posterioren Seite einen elastischen, insbesondere querelastischen, Einsatz auf, welcher die mediale und laterale Seite des Grundkörpers miteinander verbindet. Dabei ist der Einsatz bevorzugt aus einem Textillaminat gefertigt und bevorzugt über eine Nahtverbindung mit dem Grundkörper verbunden. Das Textillaminat umfasst dabei bevorzugt eine innere und äußere Gewebelage sowie eine dazwischenliegende Schaumstofflage. Der elastische Einsatz bringt den Vorteil mit sich, dass sich der im Wesentlichen unelastische Grundkörper an die Form, insbesondere an die Breite des Fußes, auf Grund dessen Querelastizität anpasst. Der Abstand der medialen und lateralen Seite des Grundkörpers zueinander kann nämlich durch diesen Einsatz variieren.

Gemäß einem weiteren Ausführungsbeispiel weist die mediale und/ oder laterale Seite des Grundkörpers der erfindungsgemäßen Sprunggelenkorthese zumindest eine erste Ausnehmung für einen Knöchelbereich eines Trägers auf. Das heißt, der Grundkörper aus dem im Wesentlichen unelastischen Textilmaterial überlappt somit nicht den Knöchel. Somit übt dieser auch keinen Druck auf den Knöchel aus.

Gemäß einem weiteren Ausführungsbeispiel weist der Grundkörper der erfindungsgemäßen Sprunggelenkorthese eine zweite Ausnehmung für eine Ferse eines Trägers auf. Dabei erstreckt sich der Grundkörper in einen Mittelfußbereich und weist ein offenes vorderes Ende auf.

Gemäß einem weiteren Ausführungsbeispiel der erfindungsgemäßen Sprunggelenkorthese weist der Grundkörper ferner einen anterioren Abschnitt auf, welcher sich bevorzugt von der lateralen Seite, insbesondere von oberhalb des Knöchels, erstreckt. Dieser anteriore Abschnitt dient dazu, die Anlagefläche des Grundkörpers weiter zu vergrößern, so dass, wie bereits zuvor erwähnt, insbesondere ein Fensterödem, durch Lücken zwischen Grundkörper und dem ersten Gurt, möglichst vermieden wird.

Gemäß einem weiteren Ausführungsbeispiel der erfindungsgemäßen Sprunggelenkorthese ist der erste Gurt aus einem elastischen, vorzugsweise semi-elastischen, und der zweite Gurt aus einem im Wesentlichen unelastischen Textilmaterial gefertigt. Dabei ist der erste Gurt bevorzugt ebenfalls aus einem Textillaminat mit einer inneren und äußeren Gewebelage sowie eine dazwischenliegenden Schaumstofflage und/ oder aus einem elastischen Webstoff gefertigt. Der zweite Gurt wiederum ist aus einem Velours gefertigt.

Gemäß einem weiteren Ausführungsbeispiel der erfindungsgemäßen Sprunggelenkorthese ist der erste und zweite Abschnitt des ersten Gurtes aus unterschiedlich elastischen Textilmaterialen gefertigt, wobei der erste Abschnitt bevorzugt eine geringere Elastizität in Längsrichtung als der zweite Abschnitt aufweist. Dabei ist der erste Abschnitt bevorzugt aus dem zuvorgenannten Textillaminat gefertigt. Der zweite Abschnitt ist wiederum aus dem elastischen Webstoff gefertigt.

Gemäß einem weiteren Ausführungsbeispiel der erfindungsgemäßen Sprunggelenkorthese erstreckt sich der erste Gurtabschnitt bis in die mediale Ausnehmung des Grundkörpers für den Knöchelbereich eines Trägers und bedeckt diese. Somit ist der Knöchel nicht vom dem Grundkörper, insbesondere von dessen unelastischen Material bedeckt, sondern vielmehr von dem bevorzugt semi-elastischen Material des ersten Gurtes. Dies bringt den Vorteil mit sich, dass der Tragekomfort der Orthese wesentlich verbessert wird, da durch diese Anordnung im Bereich des hervorstehenden Knöchels nur elastisches Material vorhanden ist, welches keine Druckstellen an dem Fuß eines Trägers erzeugt.

Gemäß einem weiteren Ausführungsbeispiel der erfindungsgemäßen Sprunggelenkorthese ist an dem Grundkörper ein streifenförmiges, vorzugsweise U-förmiges Stabilisierungselement angebracht, das den Fuß eines Trägers zumindest medial, lateral und plantar umgibt. Das Stabilisierungselement dient neben dem Grundkörper zur weiteren Stabilisierung des Fußes eines Trägers. Dabei ist dieses bevorzugt aus Kunststoff, insbesondere Polyamid, gefertigt. Das Stabilisierungselement ist bevorzugt an der Außenseite des Grundkörper angebracht, insbesondere dort aufgeschweißt, angeklebt oder besonders bevorzugt dort angenäht.

Gemäß einem weiteren Ausführungsbeispiel der erfindungsgemäßen Sprunggelenkorthese weist das Stabilisierungselement mehrere Abschnitte auf, welche derart gewinkelt zueinander angeordnet sind, dass sich das Stabilisierungselement in einem Tragezustand medial und lateral hinter einem Knöchel des Trägers bis über den Knöchel hinaus erstreckt und unterhalb einer Fußsohle eines Trägers vor dem Fersenballen in einem Mittelfußbereich zum Liegen kommt. Eine rückseitige Verbindung der beiden medial und lateral am Fuß des Trägers entlanglaufenden Schenkel ist nicht vorgesehen. Dadurch bleibt die Orthese, also der Grundkörper mit dem Stabilisierungselement, weiterhin an die Form, insbesondere an die Breite, des Fußes eines Trägers anpassbar. Der Abstand der medialen und lateralen Seite des Grundkörpers, mit den dort angebrachten Abschnitten des Stabilisierungselements, kann weiter variieren.

Gemäß einem weiteren Ausführungsbeispiel der erfindungsgemäßen Sprunggelenkorthese weist zumindest einer der Abschnitte des Stabilisierungselements, welche oberhalb oder unterhalb des Knöchels eines Trägers verlaufen, eine Ausnehmung für den Knöchel auf. Hierdurch wird sichergestellt, dass der Knöchel nicht von dem Stabilisierungselement überlagert wird, was den Tragekomfort deutlich verschlechtern würde.

Die beiden Abschnitte des Stabilisierungselements, welche oberhalb des Knöchels eines Trägers verlaufen, weisen zudem bevorzugt eine Verbreiterung auf. Dadurch wird die Anlagefläche des Stabilisierungselements vergrößert und somit auch die Stabilisierungswirkung weiter verbessert.

Schließlich bildet das Stabilisierungselement der erfindungsgemäßen Sprunggelenkorthese, gemäß einem weiteren Ausführungsbeispiel, im Übergang zwischen der plantaren Seite und der lateralen Seite des Grundköpers eine Kante aus. Diese dient dazu, ein Umknicken des Fußes zu vermeiden.

Die vorliegende Sprunggelenkorthese zeichnet sich durch eine Reihe erheblicher Vorteile aus.

Durch die erfindungsgemäße Ausbildung der Sprunggelenkorthese wird ein besonders leichtes Einsteigen in die Orthese, insbesondere in den Grundkörper, und ein besonders leichtes Positionieren der Gurte ermöglicht. Dies wird dadurch gewährleistet, da der Grundkörper der Orthese an der anterioren Seite offen ausgebildet ist. Somit kann der Träger von vorne leicht mit dem Fuß in die Sprunggelenkorthese einsteigen. Da beide Gurte der Orthese an der medialen Seite der Orthese angeordnet sind, sind diese für den Träger beim Anlegen der Orthese, insbesondere beim Anwinkeln des Beines bzw. Fußes in Richtung Oberkörper, auch sehr gut zugänglich und somit greifbar. Die eingearbeitete Positionierungshilfe für den ersten Gurt erleichtert zudem das Anlegen des ersten Gurtes. Das Umlenkelement für den zweiten Gurt erleichtert das Positionieren und Spannen des zweiten Gurtes. Durch das Umlenkelement wird nämlich der benötigte Kraftaufwand zur Erzeugung der Zugwirkung auf die Außenkante und die Bildung eines Supinationsschutzes deutlich reduziert. Auch die Anordnung der beiden Gurtenden am oberen Ende der Orthese erleichtert das Anlegen der Sprunggelenkorthese, insbesondere der Gurte, da diese für den Täger der Orthese leichter zugänglich sind.

Einen weiteren Vorteil der Erfindung stellt, neben dem leichten Einsteigen in die Orthese, insbesondere in den Grundkörper, und dem leichten Positionieren des ersten Gurtes, insbesondere durch die Positionierungshilfe, wie zuvor bereits geschrieben, der hohe Tragekomfort der Sprunggelenkorthese in einem angelegten Zustand dar. Durch den erfindungsgemäßen Aufbau der Orthese werden nämlich Einschnürungen, bspw. durch die zwei Gurte und durch das Stabilisierungselement vermieden. Dazu ist der Grundkörper bevorzugt unterhalb des ersten Gurtes angeordnet. In den Bereichen, in denen kein Grundkörper vorgesehen ist, weist der erste Gurt dafür eine besonders große Anlagefläche, also Breite, auf. Der zweite Gurt wird zudem zuletzt geschlossen und ist dabei oberhalb des ersten Gurtes angeordnet. Dieser liegt somit nicht unmittelbar auf der Haut oder dem Socken eines Trägers auf.

Zudem weist die erfindungsgemäße Sprunggelenkorthese im Vergleich zum Stand der Technik eine wesentlich bessere Stabilisierung des Sprunggelenks auf. Dies zum einen auf Grund der Stabilisierung des Sprunggelenks durch den erfindungsgemäßen Grundkörper und Verlauf des ersten Gurtes. Zum anderen aber auch auf Grund der erfindungsgemäßen Anordnung des zweiten Gurtes an dem Grundkörper, welcher einen Supinationsschutz für das Sprunggelenk des Trägers bildet. Der zweite Gurt mindert nämlich die Supinationsmöglichkeit des Fußes bzw. verhindert diese. Das Beugen und Strecken des Fußes ist dabei bevorzugt regelmäßig weiterhin möglich. Schließlich weist das Stabilisierungselement einen Umknickschutz auf, welcher insbesondere in Form einer Kante im Übergang zwischen der plantaren Seite und der lateralen Seite des Stabilisierungselements ausgebildet ist, was die Stabilisierung des Sprunggelenks ebenfalls verbessert.

Auch einen wesentlichen Vorteil der Sprunggelenkorthese stellt der Abbau bzw. die Einschränkung von Schwellungen, insbesondere von Ödemen, durch die erfindungsgemäße Orthese dar. Durch den anatomisch geformten Grundkörper in Verbindung mit dem ersten Gurt kann nämlich Kompression auf den Fuß, insbesondere auf den Spannbereich und den lateralen Knöchelbereich, ausgeübt werden. Dadurch können Schwellungen am Fuß eines Trägers reduziert und auch die Bildung von Ödemen vermieden werden. Zudem werden dadurch auch Fensterödeme vermieden. Durch die erfindungsgemäße Anordnung der mehreren Komponenten zueinander weist die Orthese nämlich keine Lücken auf und ist somit vollständig im Bereich des Spannes, des Knöchels und insbesondere der Fessel geschlossen.

Vorteilhaft ist dabei zudem, dass die erfindungsgemäße Sprunggelenkorthese einfach und somit kostengünstig in der Herstellung ist. Neben der Tatsache, dass nur wenige Komponenten für die Herstellung der Sprunggelenkorthese benötigt werden, ist insbesondere zu erwähnen, dass auch nur wenige Prozessschritte bzw. wenige unterschiedliche Fertigungsmethoden benötigt werden. Sämtliche Bauteile der Orthese, insbesondere der Grundkörper, die zwei Gurte, das Umlenkelement, das Stabilisierungselement sowie die Befestigungselemente werden nämlich durch eine Nahtverbindung miteinander verbunden.

Nachfolgend wird die Erfindung anhand mehrerer Ausführungsbeispiele und in Verbindung mit den beigefügten Zeichnungen erläutert.

Dabei zeigen:
Fiqur 1 die erfindungsgemäße Sprunggelenkorthese mit einem geschlossenen ersten und zweiten Gurt in einer dreidimensionalen Darstellung von leicht schräg vorne;
Figur 2 die Außenseite, also in einem Tragezustand die laterale Seite, der erfindungsgemäßen Sprunggelenkorthese gemäß Figur 1;
Fiqur 3 die erfindungsgemäße Sprunggelenkorthese gemäß Figur 1 von vorne, also bezogen auf einer Träger von anterior;
Figur 4 die Innenseite, also in einem Tragezustand die mediale Seite der erfindungsgemäßen Sprunggelenkorthese gemäß Figur 1;
Fiqur 5 die erfindungsgemäße Sprunggelenkorthese gemäß Figur 1 von hinten, also von der posterioren Seite;
Fiqur 6 die erfindungsgemäße Sprunggelenkorthese gemäß Figur 1 erneut von vorne, jedoch mit geöffnetem erstem und zweitem Gurt;
Figur 7 zeigt schließlich das Stabilisierungselement der erfindungsgemäßen Sprunggelenkorthese aus den vorherigen Figuren losgelöst von dem Grundkörper;

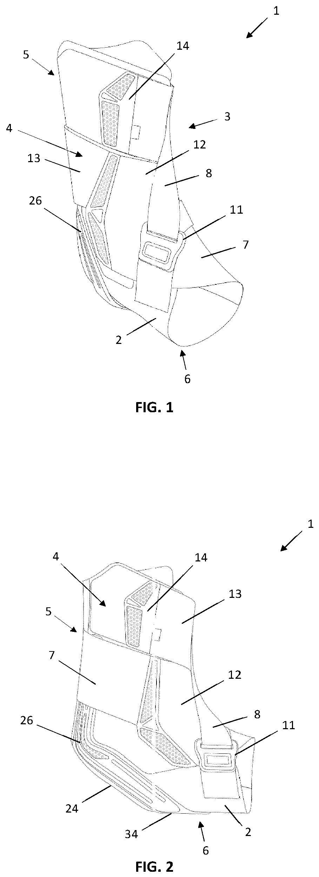

Wie in der Figur 1 zu sehen ist, weist die erfindungsgemäße Sprunggelenkorthese 1 einen Grundkörper 2 sowie einen ersten und zweiten Gurt 7, 8 auf. Der Grundkörper 2 ist dabei derart ausgebildet, dass dieser den Fuß eines Trägers medial, lateral, posterior sowie plantar umgibt. Er weist also zumindest eine mediale Seite 3, eine laterale Seite 4, eine posteriore Seite 5 sowie zumindest eine plantare Seite 6 auf. Neben den Gurten 7, 8 ist an dem Grundkörper 2 auch ein streifenförmiges, vorzugsweise U-förmiges, Stabilisierungselement 26 angebracht, das den Fuß eines Trägers medial, lateral und plantar umgibt. Die beiden Gurte 7, 8 erstrecken sich dabei von dem Grundkörper 2, insbesondere von dessen medialen Seite 3, und verlaufen dabei jeweils über den Spann eines Fußes eines Trägers. Der erste Gurt 7 ist in seiner Länge derart ausgebildet ist, dass ein erster Abschnitt 12 des Gurtes 7 sich von medial über den Spann eines Trägers nach lateral erstreckt und ein zweiter Abschnitt 13 den Fuß eines Trägers posterior und anschließend anterior umläuft, so dass sein freies Ende 14 erneut an der lateralen Seite 4 an dem Grundkörper 2 befestigbar ist. Der zweite Gurt, nämlich der Talusgurt 8, wird auf der lateralen Seite 4 des Grundkörpers 1 durch ein Umlenkelement 7 erneut über den Spann zur medialen Seite 3 umgelenkt. Mit seinem freien Ende ist er dann nach der lateralen Umlenkung ebenfalls wie der erste Gurt 7 an der medialen Seite 4 befestigbar.

Der Grundkörper 2 der erfindungsgemäßen Sprunggelenkorthese 1 ist bevorzugt aus einem im Wesentlichen unelastischen Textilmaterial gefertigt. Der erste Gurt 7 hingegen ist bevorzugt aus einem elastischen, vorzugsweise semi-elastischen, Textilmaterial gefertigt. Der zweite Gurt 8 wiederum ist aus einem Velours gefertigt. Dieses ist ein im Wesentlichen unelastisches Material. Dieses unelastische Material wird für den zweiten Gurt 8 benötigt, um den Supinationsschutz ausbilden zu können, insbesondere um die Zugkräfte auf die Außenkante des Fußes wirken zu lassen.

Bevorzugt ist der erste und zweite Abschnitt 12, 13 des ersten Gurtes 7 aus unterschiedlich elastischen Textilmaterialen gefertigt, wobei der erste Abschnitt 12 bevorzugt eine geringere Elastizität als der zweite Abschnitt 13 aufweist. Dabei ist der erste Abschnitt bevorzugt aus dem Textillaminat gefertigt. Der zweite Abschnitt wiederum ist aus einem elastischen Gewebe gefertigt. Beide Gurtabschnitte 12, 13 weisen nämlich unterschiedliche Funktionen auf. Der erste Abschnitt 12 dient dazu, wie der Grundkörper 2, den Fuß eines Trägers zu bedecken und somit zu stabilisieren, jedoch mit einer höheren Elastizität und somit Nachgiebigkeit als der Grundkörper 2, insbesondere für den Spann- und Knöchelbereich. Der zweite Abschnitt 13 dient nun dazu, die Orthese 1, insbesondere den Grundkörper 2 sowie das Stabilisierungselement 26, besonders fest an dem Fuß des Trägers zu positionieren, was durch die elastische Ausbildung des Abschnitts 13 und der damit verbundenen Möglichkeit diesen Abschnitt 13 weit aufzudehnen bzw. vorzuspannen erreicht wird. Zudem kann durch diesen Abschnitt 13 Kompression auf den Fuß eines Trägers ausgeübt werden. Dadurch können Schwellungen am Fuß eines Trägers reduziert und auch die Bildung von Ödemen vermieden werden.

In der Figur 2 ist nun die Außenseite, also in einem Tragezustand der Orthese 1 die laterale Seite 4, der erfindungsgemäßen Sprunggelenkorthese 1 gezeigt. Dabei ist zu sehen, dass der Grundkörper 2 der Sprunggelenkorthese 1 eine Ausnehmung 24 für die Ferse eines Trägers aufweist. Die Ferse wird somit nicht von der Orthese 1 bedeckt. Das Umlenkelement 11 für den zweiten Gurt 8 ist dabei in Form eines D-Ringes ausgebildet, welcher über ein Textilelement, insbesondere mittels einer Textillasche, an dem Grundkörper 2 befestigt ist. Der D-Ring weist neben einem Umlenkabschnitt ferner einen davon abstehenden Befestigungsabschnitt auf, welcher bevorzugt mit dem Umlenkabschnitt einteilig ausgebildet ist. Der Befestigungsabschnitt ist in diesem Ausführungsbeispiel an der Lasche angenäht. Alternativ kann er auch direkt an dem Grundkörper 2 angenäht werden. Hierzu weist dieser vorzugsweise einen nut-förmigen Rücksprung auf. Material und Materialstärke des Befestigungsabschnittes sind nun im Bereich des Rücksprungs bevorzugt so gewählt, dass dieser von einer Nähmaschine, insbesondere von deren Nähnadel, durchdringbar ist, so dass das Umlenkelement 11 über einen Faden im Bereich des Rücksprungs mit der Lasche oder dem Grundkörper 2 verbindbar ist.

Der erste Gurt 7, insbesondere der erste Gurtabschnitt 12, erstreckt sich, wie in Figur 2 zu sehen ist, von der medialen Seite des Grundkörpers 2 auf Höhe des Innenknöchels über den Spann zur lateralen Seite 4 des Grundkörpers 2 oberhalb des Außenknöchels. Von dort aus umläuft der zweite Abschnitt 13 des ersten Gurtes 7 die Außenseite des Grundkörpers 2 und somit den Fuß eines Trägers, bevorzugt auf Höhe der Fessel, vollständig, also auch an der posterioren Seite 5, und wird mit seinem freien Ende 14 an der lateralen Seite 4 an dem Grundkörper 2 und zwar oberhalb des dort verlaufenden ersten Gurtabschnitts 12 befestigt.

Das an dem Grundkörper 2 angebrachte Stabilisierungselement 26 der Sprunggelenkorthese 1, welches, wie zu sehen ist, unterhalb des ersten Gurtes 7 an dem Grundkörper 2 entlang verläuft, bildet im Übergang zwischen der plantaren Seite 6 und der lateralen Seite 4 des Grundköpers 2 eine Kante 34 aus. Diese dient dazu, ein Umknicken des Fußes möglichst zu vermeiden.

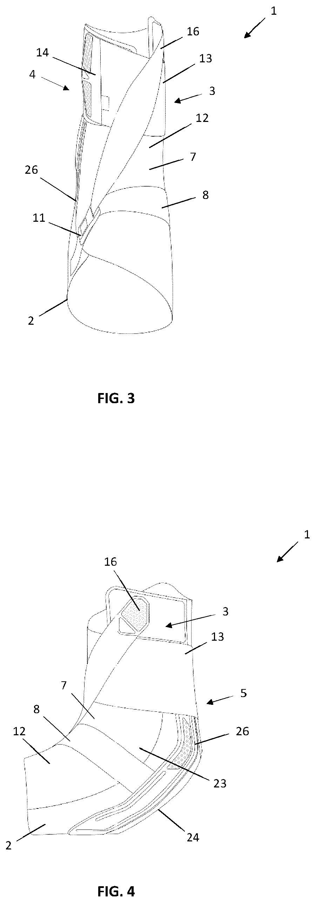

Figur 3 zeigt nun die erfindungsgemäße Sprunggelenkorthese 1 gemäß Figur 1 von vorne, also bezogen auf den Träger von anterior. Wie zu sehen ist, weist der Grundkörper 2 mit dem Stabilisierungselement 26 ein offenes unteres Ende auf, so dass der Vorfußbereich, insbesondere ein Zehenbereich, nicht von dem Grundköper 2 bedeckt ist. Der Grundkörper 2 erstreckt sich also nur bis in einen Mittelfußbereich. Dadurch ist die Bewegungsfreiheit der Zehen nicht eingeschränkt, so dass die Abrollbewegung des Fußes eines Trägers bei einer Fortbewegung nicht negativ beeinträchtigt wird. Aus der Figur 3 ist der Verlauf der beiden Gurte 7, 8 um das Sprunggelenk gut zu entnehmen. Wie bereits zuvor beschrieben, beginnt dabei der erste Gurt 7, insbesondere dessen erster Gurtabschnitt 12, an der medialen Seite 3 des Grundkörpers 2 und verläuft dann über den Spann zur lateralen Seite 4. Von dort aus umläuft der zweite Abschnitt 13 des ersten Gurtes 7 die Außenseite des Grundkörpers 2 auf Höhe der Fessel vollständig und wird dann mit seinem freien Ende 14 an der lateralen Seite 4 an dem Grundkörper 2 oberhalb des dort verlaufenden ersten Gurtabschnitts 12 befestigt. Der zweite Gurt 8, insbesondere der Talusgurt, erstreckt sich dabei ebenfalls von der medialen Seite 3 des Grundkörpers 2 über den Spann des Fußes eines Trägers. Er wird dann auf der lateralen Seite 4 des Grundkörpers 2 in einem Mittelfußbereich über ein an dem Grundkörper 2 angebrachtes Umlenkelement 11 umgelenkt. Nach der Umlenkung verläuft dieser erneut über den Spann und wird dann auf der medialen Seite 3 an dem Grundkörper 2 befestigt. Somit sind sowohl das freie Ende 14 des ersten Gurtes 7, als auch das freie Ende 16 des zweiten Gurtes 8, oberhalb des Innen- und Außenknöchels, also an der medialen Seite 3 und an der lateralen Seite 4, an dem Grundkörper 2, insbesondere an dessen oberen Ende, befestigt. Dies erleichtert das Anlegen der Sprunggelenkorthese 1, insbesondere das Greifen und Positionieren der Gurte 7, 8, wesentlich, da diese für den Täger der Orthese 1, auf Grund der näheren Anordnung zur Körpermitte, leichter zugänglich sind. Dies insbesondere beim dortigen Positionieren der Gurte 7, 8, aber auch beim Lösen der Gurte 7, 8.

Figur 4 zeigt die Innenseite, also in einem Tragezustand die mediale Seite 3 der erfindungsgemäßen Sprunggelenkorthese 1. Zu sehen ist, dass der Grundkörper 2 mit dem daran angeordneten Stabilisierungselement 26 an der medialen Seite 3 eine erste Ausnehmung 23 für einen Knöchelbereich eines Trägers aufweist. Der Grundkörper 2 aus dem im Wesentlichen unelastischen Textilmaterial überlappt somit nicht den Knöchel eines Trägers. Somit kann dieser auch keinen unangenehmen Druck auf den Knöchel ausüben. Vielmehr erstreckt sich der erste Gurt 7, insbesondere dessen erster elastischer Abschnitt 12, über den Knöchelbereich sowie über die Ausnehmung 23. Zu sehen ist ebenfalls erneut die zweite Ausnehmung 24 des Grundkörpers 2, nämlich die für eine Ferse. Die Ferse wird somit nicht von der Orthese 1 bedeckt.

Auch aus der Figur 3 ist der Verlauf der beiden Gurte 7, 8 gut zu erkennen. Dabei ist der erste Abschnitt 12 des ersten Gurtes 7 sichtbar, welcher an der medialen Seite 3 an dem Grundkörper 2 befestigt ist. Zudem ist auch der zweite Abschnitt 13 gezeigt, zumindest der mediale Teil, welcher den Fuß eines Trägers vollständig, also auch an der posterioren Seite 5, umläuft. Von dem zweiten Gurt 8 ist zum einen der untere Abschnitt, welcher sich von der medialen Seite 3 wegerstreckt, aber auch der umgelenkte obere Abschnitt sichtbar. Zudem ist sein freies Ende 16 gezeigt, wodurch der Gurt 8 an dem Grundköper 2 befestigt wird.

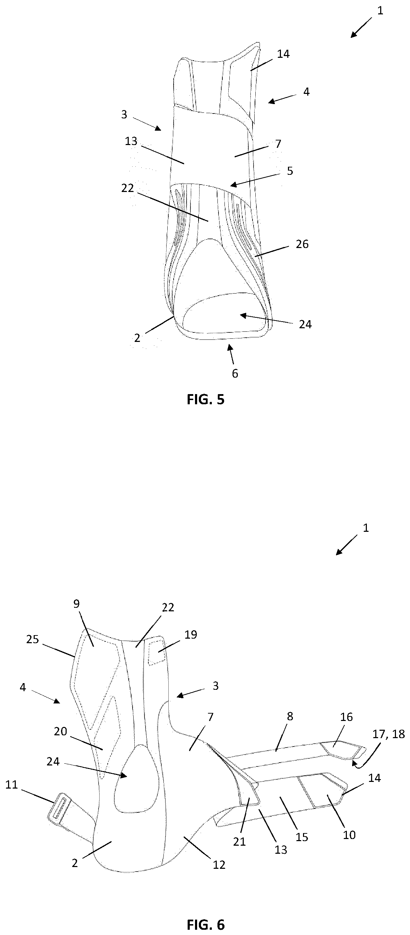

Figur 5 zeigt schließlich die erfindungsgemäße Sprunggelenkorthese 1 von hinten, also von der posterioren Seite 5. Dabei ist neben der Öffnung 24 für die Ferse eines Trägers des Grundkörpers 2, insbesondere der elastische Einsatz 22 zu sehen. Der Einsatz 22 erstreckt sich dabei von der plantaren Seite 6, also von der Öffnung 24 bis an das obere Ende der Orthese 1 und ist dabei zwischen dem Stabilisierungselement 26 angeordnet. Dadurch, insbesondere durch die Querelastizität des Einsatzes 22, ist es möglich, dass der Abstand zwischen der medialen Seite 3 und lateralen Seite 4 des Grundkörpers 2 variiert, so dass die Orthese 1 an den Fuß eines Trägers anpassbar ist. Zudem ist der erste Gurt 7, insbesondere dessen zweiter Abschnitt 13, zu sehen, welcher sich an der Rückseite der Orthese 1 von lateral nach medial erstreckt, also den Fuß eines Trägers posterior umläuft. Das freie Ende 14 des Gurtes wird dabei, also nach dem der Gurt 7 von lateral nach medial verläuft, an der lateralen Seite 4 des Grundkörpers 2 befestigt. Dies insbesondere an dem oberen Ende des Grundkörper 2. Das erleichtert das Anlegen der Sprunggelenkorthese 1.

Figur 6 zeigt nun schließlich die erfindungsgemäße Sprunggelenkorthese 1 erneut von vorne, jedoch mit einem geöffnetem ersten und zweiten Gurt 7, 8, also in einem Zustand, bevor diese an einen Fuß eines Trägers angelegt wird. Der Grundköper 2 weist neben dem Einsatz 22 und der Ausnehmung 24 für die Ferse eines Trägers einen anterioren Abschnitt 25 auf, welcher sich von der lateralen Seite 4, insbesondere von oberhalb des Knöchels, erstreckt. Dieser anteriore Abschnitt 25 dient dazu, die Anlagefläche des Grundkörpers 2 weiter zu vergrößern, so dass wie bereits zuvor erwähnt, ein Fensterödem, durch Lücken zwischen Grundkörper 2 und dem ersten Gurt 7, möglichst vermieden wird. Durch die auf der anterioren Seite jedoch weitestgehend offene Ausbildung des Grundköpers 2 und die einseitige Anordnung der Gurte 7, 8, wird ein besonders leichtes Einsteigen in die Orthese 1, insbesondere in den Grundkörper 2, ermöglicht.

Die beiden Gurte 7, 8 sind an der medialen Seite 3 an dem Grundkörper 2 befestigt. Dadurch sind diese für den Träger beim Anlegen der Orthese 1 auch sehr gut zugänglich und greifbar. Nachdem der Träger der Orthese 1 in den Grundkörper 2 eingestiegen ist, legt er den ersten Gurt 7 an. Dabei führt er den Gurt 7 zunächst auf Höhe des Innenknöchels über den Spann zur lateralen Seite 4 des Grundkörpers 2 oberhalb des Außenknöchels. Dort befestigt er den ersten Gurt 7, insbesondere dessen ersten Abschnitt 12. Hierfür weist der Grundkörper 2 an der lateralen Seite 4 ein fünftes Befestigungselement 20 und der erste Gurt 7 an seiner Innenseite 15 an dem Ende des ersten Gurtabschnitts 13 ein sechstes Befestigungselement 21 auf. Von dort aus führt der Träger den zweiten Abschnitt 13 des ersten Gurtes 7 an der Außenseite des Grundkörpers 2 posterior um den Fuß eines Trägers entlang, insbesondere auf Höhe der Fessel, und befestigt das freie Ende 14 dann an der lateralen Seite 4, insbesondere an einem an dem Grundkörper 2 oberhalb des dort verlaufenden ersten Gurtabschnitts 12 angeordneten ersten Befestigungselement 9. Dazu weist der Gurt 7 an der Innenseite 15 an seinem freien Ende 14 einen zweiten Befestigungsabschnitt 10 auf. Hierdurch wird das Sprunggelenk stabilisiert. Es wird aber auch der Abbau bzw. die Einschränkung von Schwellungen, insbesondere von Ödemen, unterstütz. Durch den ersten Gurt 7 wird nämlich Kompression auf den Fuß ausgeübt.

Nachdem der Grundkörper 2 und der erste Gurt 7 angelegt wurde, wird der zweite Gurt 8 von der medialen Seite 3 von unterhalb des Innenknöchels über den Spann des Fußes eines Trägers geführt. Dort wird er in einem Mittelfußbereich über ein an dem Grundkörper 2 angebrachtes Umlenkelement 11 umgelenkt. Nach der Umlenkung verläuft dieser von der lateralen Seite 4 von unterhalb des Außenknöchels erneut über den Spann und wird dann auf der medialen Seite 3 an dem Grundkörper 2 oberhalb des Innenknöchels im Bereich der Fessel an einem vierten Befestigungselement 19 befestigt. Hierzu ist an der Außenseite 17 des zweiten Gurtes 8 an dessen freien Ende 16 ein drittes Befestigungselement 18 vorgesehen. Wie bereits erläutert, bringt der Träger durch den zweiten Gurt 8 eine Zugwirkung auf die Außenkante des Fußes auf. Dadurch wird ein Supinationsschutz für das Sprunggelenk des Trägers gebildet.

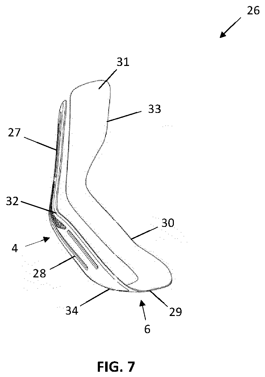

Figur 7 zeigt schließlich das Stabilisierungselement 26 der Sprunggelenkorthese 1 aus den vorherigen Figuren losgelöst von dem Grundkörper 2. Wie zu sehen ist, weist das streifenförmige, vorzugsweise U-förmige, Stabilisierungselement 26 mehrere Abschnitte 27, 28, 29, 30, 31 auf, welche derart gewinkelt zueinander angeordnet sind, dass sich das Stabilisierungselement 26 in einem Tragezustand medial und lateral hinter einem Knöchel des Trägers bis über den Knöchel hinaus erstreckt und unter einer Fußsohle eines Trägers vor dem Fersenballen in einem Mittelfußbereich zum Liegen kommt. Eine rückseitige Verbindung der beiden medial und lateral am Fuß des Trägers entlanglaufenden Abschnitte 27, 31 ist nicht vorhanden. Dadurch bleibt die Orthese 1, also der Grundkörper 2 mit dem Stabilisierungselement 26, weiterhin an die Form, insbesondere an die Breite, des Fußes eines Trägers anpassbar.

Ferner weist der mediale Abschnitt 31, welcher oberhalb des Knöchels eines Trägers verläuft, bevorzugt eine Verbreiterung 33 auf. Dadurch wird die Anlagefläche des Stabilisierungselements 26 vergrößert und somit auch die Stabilisierungswirkung weiter verbessert. Zudem weist bevorzugt der Abschnitt 28 des Stabilisierungselements 26, welcher unterhalb des Außenknöchels eines Trägers verläuft, eine Ausnehmung 32 für den Knöchel auf. Hierdurch wird sichergestellt, dass der Knöchel nicht von dem Stabilisierungselement 26 überlagert wird, was den Tragekomfort deutlich verschlechtern würde. Wie bereits in Figur 2 gezeigt und dazu beschrieben, weist das Stabilisierungselement 26 im Übergang zwischen der plantaren Seite 6 und der lateralen Seite 4 des Grundköpers 2 eine Kante 34 auf, welche als Umknickschutz dient.

Der Schutzumfang wird durch die nachfolgenden Ansprüche definiert.

## Patentansprüche

1. Sprunggelenkorthese (1), bestehend aus einem Grundkörper (2), der den Fuß eines Trägers zumindest medial, lateral und posterior sowie plantar umgibt, sowie zumindest einem ersten Gurt (7), der sich von der medialen Seite (3) des Grundkörpers (1) erstreckt und zumindest anterior über den Fuß des Trägers verläuft, wobei der Grundkörper (1) an der lateralen Seite (4) zumindest ein erstes Befestigungselement (9) für den ersten Gurt (7) aufweist und wobei der erste Gurt (7) in seiner Länge derart ausgebildet ist, dass ein erster Abschnitt (12) des Gurtes (7) sich von medial nach lateral erstreckt und ein zweiter Abschnitt (13) den Fuß eines Trägers posterior umläuft, so dass ein an seinem freien Ende (14) an der Innenseite (15) angebrachtes zweites Befestigungselement (10) an dem ersten lateralen Befestigungselement (9) des Grundkörpers (1) befestigbar ist, **dadurch gekennzeichnet, dass** zumindest ein zweiter Gurt (8), der aus einem im Wesentlichen unelastischen Textilmaterial besteht, sich von der medialen Seite (3) des Grundkörpers (1) erstreckt und zumindest anterior über den Fuß des Trägers verläuft, **dass** der Grundkörper (1) an der lateralen Seite (4) zumindest ein Umlenkelement (11) für den zweiten Gurt (8) aufweist, und **dass** der zweite Gurt (8) an seinem freien Ende (16) an der Außenseite (17) zumindest ein drittes Befestigungselement (18) aufweist, so dass dieser nach einer lateralen Umlenkung an der medialen Seite (3) über ein viertes Befestigungselement (19) auf sich selbst, an dem Grundkörper (1) oder an dem ersten Gurt (7) befestigbar ist.

2. Sprunggelenkorthese (1) nach Anspruch 1, **dadurch gekennzeichnet, dass** der Grundkörper (2) an der lateralen Seite (4) ein fünftes Befestigungselement (20) und der erste Gurt (7) an seiner Innenseite (15) an dem Ende des ersten Gurtabschnitts (12) ein sechstes Befestigungselement (21) aufweist, so dass der erste Gurt (7) an der lateralen Seite (4) des Grundköpers (2) befestigbar ist, bevor dieser den Fuß eines Trägers posterior umläuft und erneut lateral an dem ersten Befestigungselement (9) an dem Grundkörper (2) befestigtbar ist.

3. Sprunggelenkorthese (1) nach Anspruch 1 oder 2, **dadurch gekennzeichnet, dass** der Grundkörper (2) aus einem im Wesentlichen unelastischen Textilmaterial gefertigt ist.

4. Sprunggelenkorthese (1) nach einem der vorherigen Ansprüche, **dadurch gekennzeichnet, dass** der Grundkörper (2) an der posterioren Seite (5) einen elastischen Einsatz (22) aufweist, welcher die mediale und laterale Seite (3, 4) des Grundkörpers (2) miteinander verbindet.

5. Sprunggelenkorthese (1) nach einem der vorherigen Ansprüche, **dadurch gekennzeichnet, dass** die mediale und/ oder laterale Seite (3, 4) des Grundkörpers (2) zumindest eine erste Ausnehmung (23) für einen Knöchelbereich eines Trägers aufweist.

6. Sprunggelenkorthese (1) nach einem der vorherigen Ansprüche, **dadurch gekennzeichnet, dass** der Grundkörper (2) eine zweite Ausnehmung (24) für eine Ferse eines Trägers aufweist.

7. Sprunggelenkorthese (1) nach einem der vorherigen Ansprüche, **dadurch gekennzeichnet, dass** der Grundkörper (2) ferner einen anterioren Abschnitt (25) aufweist, welcher sich bevorzugt von der lateralen Seite (4), insbesondere von oberhalb des Knöchels, erstreckt.

8. Sprunggelenkorthese (1) nach einem der vorherigen Ansprüche, **dadurch gekennzeichnet, dass** der erste Gurt (7) aus einem elastischen Textilmaterial gefertigt ist.

9. Sprunggelenkorthese (1) nach einem der vorherigen Ansprüche, **dadurch gekennzeichnet, dass** der erste und zweite Abschnitt (12, 13) des ersten Gurtes (7) aus unterschiedlich elastischen Textilmaterialen gefertigt ist, wobei der erste Abschnitt (12) bevorzugt eine geringe Elastizität in Längsrichtung als der zweite Abschnitt (13) aufweist.

10. Sprunggelenkorthese (1) nach einem der vorherigen Ansprüche, **dadurch gekennzeichnet, dass** der erste Gurtabschnitt (12) sich bis in die mediale Ausnehmung (23) des Grundkörpers (2) für einen Knöchelbereich eines Trägers erstreckt und diesen bedeckt.

11. Sprunggelenkorthese (1) nach einem der vorherigen Ansprüche, **dadurch gekennzeichnet, dass** an dem Grundkörper (2) ein streifenförmiges, vorzugsweise U-förmiges, Stabilisierungselement (26) angebracht ist, das den Fuß eines Trägers zumindest medial, lateral und plantar umgibt.

12. Sprunggelenkorthese (1) nach Anspruch 11, **dadurch gekennzeichnet, dass** das Stabilisierungselement (26) mehrere Abschnitte (27, 28, 29, 30, 31) aufweist, welche derart gewinkelt zueinander angeordnet sind, dass sich das Stabilisierungselement (26) in einem Tragezustand medial und lateral hinter einem Knöchel des Trägers bis über den Knöchel hinaus erstreckt und unter einer Fußsohle eines Trägers vor dem Fersenballen in einem Mittelfußbereich zum Liegen kommt.

13. Sprunggelenkorthese (1) nach Anspruch 11 oder 12, **dadurch gekennzeichnet, dass** zumindest einer der Abschnitte (28) des Stabilisierungselements (26), welche oberhalb oder unterhalb des Knöchels eines Trägers verlaufen, eine Ausnehmung (32) für den Knöchel aufweist.

14. Sprunggelenkorthese (1) nach einem der Ansprüche 11 bis 13, **dadurch gekennzeichnet, dass** zumindest einer der Abschnitte (27, 31) des Stabilisierungselements (26), welche oberhalb des Knöchels eines Trägers verlaufen, eine Verbreiterung (33) aufweist.

15. Sprunggelenkorthese (1) nach einem der Ansprüche 11 bis 14, **dadurch gekennzeichnet, dass** das Stabilisierungselement (26) im Übergang zwischen der plantaren Seite (6) und der lateralen Seite (4) des Grundköpers (2) eine Kante (34) ausbildet, die dazu dient, ein Umknicken des Fußes zu vermeiden.

## Claims

1. An ankle joint brace (1) consisting of a main body (2) that surrounds the foot of the wearer at least medially, laterally and posteriorly as well as plantarly, and at least one first strap (7), which extends from the medial side (3) of the main body (1) and runs at least anteriorly over the foot of the wearer, wherein the main body (1) on the lateral side (4) has at least one first fastening element (9) for the first strap (7), and wherein the first strap (7) is designed in such a way in terms of its length that a first portion (12) of the strap (7) extends from medial to lateral and a second portion (13) wraps around the foot of a wearer posteriorly, so that a second fastening element (10) attached to its free end (14) on the inner side (15) can be fastened to the first lateral fastening element (9) of the main body (1), **characterized in that** at least one second strap (8), which consists of a substantially inelastic textile material, extends from the medial side (3) of the main body (1) and runs at least anteriorly over the foot of the wearer, **in that** the main body (1) on the lateral side (4) has at least one deflection element (11) for the second strap (8), and **in that** the second strap (8) has at least one third fastening element (18) on its free end (16) on the outer side (17), so that after a lateral deflection on the medial side (3) the at least one third fastening element can be fastened to itself, to the main body (1) or to the first strap (7) via a fourth fastening element (19).

2. The ankle joint brace (1) according to claim 1, **characterised in that** the main body (2) has a fifth fastening element (20) on the lateral side (4) and the first strap (7) has a sixth fastening element (21) on its inner side (15) at the end of the first strap portion (12), so that the first strap (7) can be fastened to the lateral side (4) of the main body (2) before it wraps posteriorly around the foot of a wearer and can be fastened again laterally to the first fastening element (9) on the main body (2).

3. The ankle joint brace (1) according to claim 1 or 2, **characterised in that** the main body (2) is made of a substantially inelastic textile material.

4. The ankle joint brace (1) according to any one of the preceding claims, **characterised in that** the main body (2) has an elastic insert (22) on the posterior side (5), which insert connects the medial and lateral sides (3, 4) of the main body (2) to each other.

5. The ankle joint brace (1) according to any one of the preceding claims, **characterised in that** the medial and/or lateral side (3, 4) of the main body (2) has at least one first recess (23) for an ankle area of a wearer.

6. The ankle joint brace (1) according to any one of the preceding claims, **characterised in that** the main body (2) has a second recess (24) for a heel of a wearer.

7. The ankle joint brace (1) according to any one of the preceding claims, **characterised in that** the main body (2) further has an anterior portion (25) which preferably extends from the lateral side (4), in particular from above the ankle.

8. The ankle joint brace (1) according to any one of the preceding claims, **characterised in that** the first strap (7) is made of an elastic textile material.

9. The ankle joint brace (1) according to one of the preceding claims, **characterised in that** the first and second portions (12, 13) of the first strap (7) are made of textile materials with different elasticities, wherein the first portion (12) preferably has a lower elasticity in the longitudinal direction than the second portion (13).

10. The ankle joint brace (1) according to any one of the preceding claims, **characterised in that** the first strap portion (12) extends into the medial recess (23) of the main body (2) for an ankle area of a wearer and covers it.

11. The ankle joint brace (1) according to any one of the preceding claims, **characterised in that** a strip-shaped, preferably U-shaped stabilising element (26) is attached to the main body (2) and surrounds the foot of a wearer at least medially, laterally and plantarly.

12. The ankle joint brace (1) according to claim 11, **characterised in that** the stabilising element (26) has several portions (27, 28, 29, 30, 31) which are arranged at an angle to each other such that, when worn, the stabilising element (26) extends medially and laterally behind an ankle of the wearer, beyond the ankle, and comes to rest under the sole of the foot of a wearer in front of the heel pad in a midfoot area.

13. The ankle joint brace (1) according to claim 11 or 12, **characterised in that** at least one of the portions (28) of the stabilising element (26) which run above or below the ankle of a wearer has a recess (32) for the ankle.

14. The ankle joint brace (1) according to any one of claims 11 to 13, **characterised in that** at least one of the portions (27, 31) of the stabilising element (26) which run above the ankle of a wearer has a widening (33).

15. The ankle joint brace (1) according to any one of claims 11 to 14, **characterised in that** the stabilising element (26) forms an edge (34) in the transition between the plantar side (6) and the lateral side (4) of the main body (2), which edge serves to prevent the foot from twisting.

## Revendications

1. Orthèse de cheville (1), constituée d'un corps de base (2), qui entoure au moins médialement, latéralement et postérieurement, ainsi que du côté plantaire le pied d'un porteur, ainsi que d'au moins une première sangle (7), qui s'étend à partir de la face (3) médiale du corps de base (1) et s'écoule au moins antérieurement par-dessus le pied du porteur, sur la face (4) latérale, le corps de base (1) comportant au moins un premier élément de fixation (9) pour la première sangle (7) et dans sa longueur, la première sangle (7) étant constituée de telle sorte qu'un premier segment (12) de la sangle (7) s'étende du côté médial vers le côté latéral et qu'un deuxième segment (13) entoure postérieurement le pied d'un porteur, de telle sorte qu'un deuxième élément de fixation (10) monté sur son extrémité (14) libre sur la face intérieure (15) puisse se fixer sur le premier élément de fixation (9) latéral du corps de base (1), **caractérisée en ce qu'**au moins une deuxième sangle (8), qui est constituée d'une matière textile sensiblement non élastique s'étend à partir de la face (3) médiale du corps de base (1) et s'écoule au moins antérieurement par-dessus le pied du porteur, **en ce que** sur la face (4) latérale, le corps de base (1) comporte au moins un élément de renvoi (11) pour la deuxième sangle (8) et **en ce que** la deuxième sangle (8) comporte sur son extrémité (16) libre sur la face extérieure (17) au moins un troisième élément de fixation (18), de telle sorte qu'après un renvoi latéral sur la face (3) médiale, celui-ci puisse se fixer par l'intermédiaire d'un quatrième élément de fixation (19) sur lui-même, sur le corps de base (1) ou sur la première sangle (7).

2. Orthèse de cheville (1) selon la revendication 1, **caractérisée en ce que** le corps de base (2) comporte sur la face (4) latérale un cinquième élément de fixation (20) et **en ce que** la première sangle (7) comporte sur sa face intérieure (15), sur l'extrémité du premier segment (12) de courroie un sixième élément de fixation (21), de telle sorte que la première sangle (7) puisse se fixer sur la face (4) latérale du corps de base (2), avant que celui-ci n'entoure postérieurement le pied d'un porteur et puisse se fixer à nouveau latéralement sur le premier élément de fixation (9) sur le corps de base (2).

3. Orthèse de cheville (1) selon la revendication 1 ou 2, **caractérisée en ce que** le corps de base (2) est fabriqué en une matière textile sensiblement non élastique.

4. Orthèse de cheville (1) selon l'une quelconque des revendications précédentes, **caractérisée en ce que** le corps de base (2) comporte sur la face (5) postérieure un insert (22) élastique, lequel assemble l'une avec l'autre la face (3, 4) médiale et latérale du corps de base (2).

5. Orthèse de cheville (1) selon l'une quelconque des revendications précédentes, **caractérisée en ce que** la face (3, 4) médiale et/ou latérale du corps de base (2) comporte au moins un premier évidement (23) pour une zone malléolaire d'un porteur.

6. Orthèse de cheville (1) selon l'une quelconque des revendications précédentes, **caractérisée en ce que** le corps de base (2) comporte un deuxième évidement (24) pour un talon d'un porteur.

7. Orthèse de cheville (1) selon l'une quelconque des revendications précédentes, **caractérisée en ce que** le corps de base (2) comporte par ailleurs un segment (25) antérieur, lequel s'étend de préférence à partir de la face (4) latérale, notamment à partir d'au-dessus de la malléole.

8. Orthèse de cheville (1) selon l'une quelconque des revendications précédentes, **caractérisée en ce que** la première sangle (7) est fabriquée dans une matière textile élastique.

9. Orthèse de cheville (1) selon l'une quelconque des revendications précédentes, **caractérisée en ce que** le premier et le deuxième segments (12, 13) de la première sangle (7) sont fabriqués dans des matières textile d'élasticité différente, dans la direction longitudinale, le premier segment (12) faisant preuve de préférence d'une élasticité inférieure à celle du deuxième segment (13).

10. Orthèse de cheville (1) selon l'une quelconque des revendications précédentes, **caractérisée en ce que** le premier segment (12) de sangle s'étend jusque dans l'évidement (23) médial du corps de base (2) pour une zone malléolaire d'un porteur et recouvre celle-ci.

11. Orthèse de cheville (1) selon l'une quelconque des revendications précédentes, **caractérisée en ce que** sur le corps de base (2) est monté un élément de stabilisation (26) en forme de bande, de préférence en forme de U qui entoure le pied d'un porteur au moins médialement, latéralement et du côté plantaire.

12. Orthèse de cheville (1) selon la revendication 11, **caractérisée en ce que** l'élément de stabilisation (26) comporte plusieurs segments (27, 28, 29, 30, 31), lesquels sont placés de manière coudée les uns par rapport aux autres de telle sorte qu'en situation portée, médialement et latéralement derrière une malléole d'un porteur, l'élément de stabilisation (26) s'étende jusqu'au-delà de la malléole et sous une plante de pied d'un porteur, vienne se situer à l'avant du coussinet du talon, sur le médio-pied.

13. Orthèse de cheville (1) selon la revendication 11 ou 12, **caractérisée en ce qu'**au moins l'un des segments (28) de l'élément de stabilisation (26), lesquels s'écoulent au-dessus ou en-dessous de la malléole d'un porteur comporte un évidement (32) pour la malléole.

14. Orthèse de cheville (1) selon l'une quelconque des revendications 11 à 13, **caractérisée en ce qu'**au moins l'un des segments (27, 31) de l'élément de stabilisation (26), lesquels s'écoulent au-dessus de la malléole d'un porteur comporte un élargissement (33).

15. Orthèse de cheville (1) selon l'une quelconque des revendications 11 à 14, **caractérisée en ce qu'**à la transition entre la face (6) plantaire et la face (4) latérale du corps de base (2), l'élément de stabilisation (26) constitue une arête (34) qui sert à empêcher une distorsion du pied.
